(19) Europäisches Patentamt
European Patent Office
Office européen des brevets

(11) Publication number: 0 055 602
A2

# (12) EUROPEAN PATENT APPLICATION

(21) Application number: 81306075.3

(22) Date of filing: 23.12.81

(51) Int. Cl.[3]: C 07 D 519/04
C 07 C 149/14, A 61 K 31/395

(30) Priority: 29.12.80 US 220472

(43) Date of publication of application: 07.07.82 Bulletin 82/27

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Gerzon, Koert
61, Kessler Boulevard East Drive
Indianapolis Indiana 46220(US)

(72) Inventor: Miller, Jean Corinne
5808 Central Avenue
Indianapolis Indiana 46220(US)

(74) Representative: Crowther, Terence Roger et al,
Erl Wood Manor
Windlesham Surrey GU20 6PH(GB)

## (54) Oxazolidinedione sulfide compounds.

(57) Thioethyl derivatives of dimeric indole-dihydroindole oxazolidinediones are antimitotic agents and have antitumor activity in mammals.

EP 0 055 602 A2

# Oxazolidinedione Sulfide Compounds

This invention concerns a class of novel oxazolidinedione sulfide compounds which have been discovered to be anti-tumor agents in mammals.

United States patent 4,096,148 describes various unsubstituted or substituted 3-spiro-5''-oxazolidine-2'',4''-dione derivatives of VLB, desacetyl VLB and 4-desacetyl vincristine and their preparation. These oxazolidinediones are prepared by reaction of, for example, vinblastine with an isocyanate RNCO (where R is other than H). One of the compounds thus prepared is 3''-(2-chloroethyl)-3-spiro-5''-oxazolidine-2'',4''-dione. This compound has proved to be an extremely active anti-neoplastic agent but is also described in the aforesaid patent as being useful as an intermediate--see Col. 7 lines 49-58--for the preparation of the corresponding 3''-(2-thioethyl) derivative.

It has now been discovered that a pharmaceutical formulation which comprises as active ingredient a thioethane of formula I

$$Q-CH_2-CH_2-S-R^6 \quad I$$

wherein Q is a 3"-oxazolidinedionyl radical of the formula II:

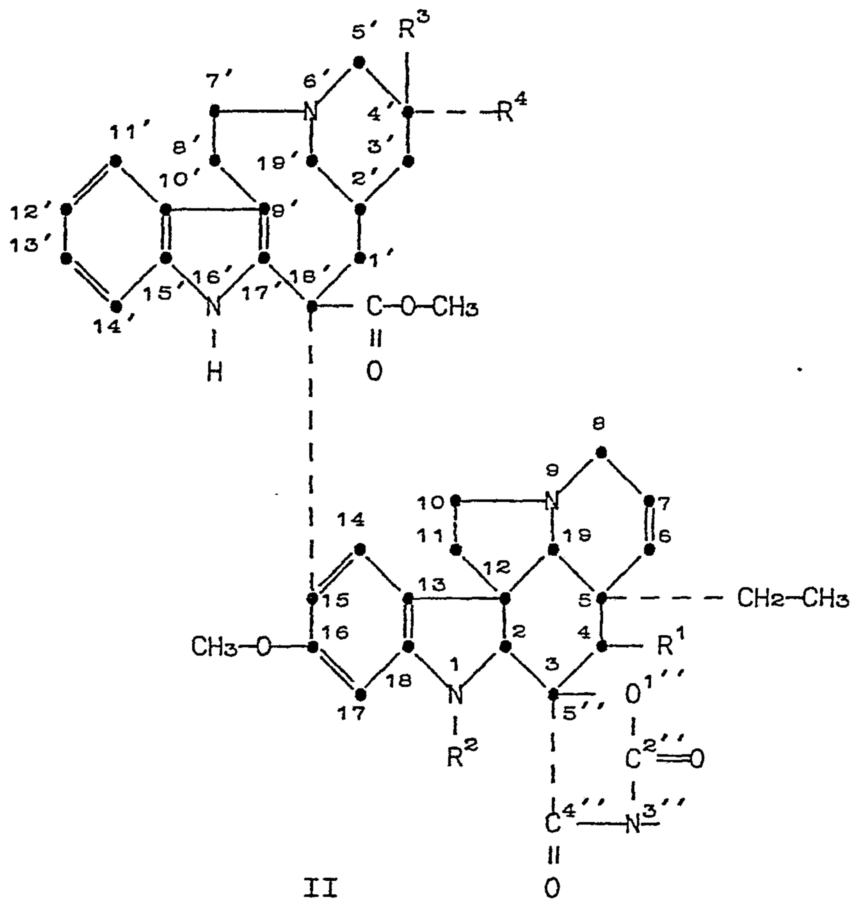

II

wherein $R^1$ is -OH or $-O-CO-CH_3$; $R^2$ is CHO or $CH_3$; one of $R^3$ and $R^4$ is OH or H and the other is ethyl; and

$R^6$ is H, $CH_3$ or $S-CH_2-CH_2-Z$;

Z is Q, NCO, or $NH-CO_2-(C_1-C_3)$alkyl, provided that when Z is NCO, $R^1$ is $-O-CO-CH_3$;
or a pharmaceutically-acceptable acid addition salt thereof associated with at least one carrier or diluent therefor is an anti-tumor agent in mammals.

Preferred thioethanes of formula V include:

vinblastine 3''-(β-thioethyl)-3-spiro-5''-oxazolidine-2'',4''-dione, sulfate salt,

vinblastine 3''-(β-[β-(methoxycarbonylamino)-ethyldithio]ethyl)-3-spiro-5''-oxazolidine-2'',4''-dione,

bis-[β-(vinblastine 3-spiro-5''-oxazolidinone-2'',4''-dione-3''-yl)ethyl]disulfide,

vinblastine 3''-(β-methylmercaptoethyl)-3-spiro-5''-oxazolidine-2'',4''-dione, and

vinblastine 3''-(β-[β-isocyanatoethyldithio]-ethyl)-3-spiro-5''-oxazolidine-2'',4''-dione.

There has also been discovered an isocyanate of the formula

$OCN-CH_2-CH_2-S-R^7$ wherein $R^7$ is $CH_3$ or $S-CH_2-CH_2-NCO$ which is useful as an intermediate in the preparation of thioethyl compounds of formula I.

Thioethyl compounds of formula I as before defined, excluding those free base compounds wherein $R^6$ is H, are novel.

There has also been discovered a process for preparing a β-thioethyl compound of formula I as before defined wherein Q, $R^1$, $R^2$, $R^3$, $R^4$, $R^6$ and Z as before defined provided that when Z is NCO, $R^1$ is $-O-CO-CH_3$; which comprises reacting a compound of the formula III

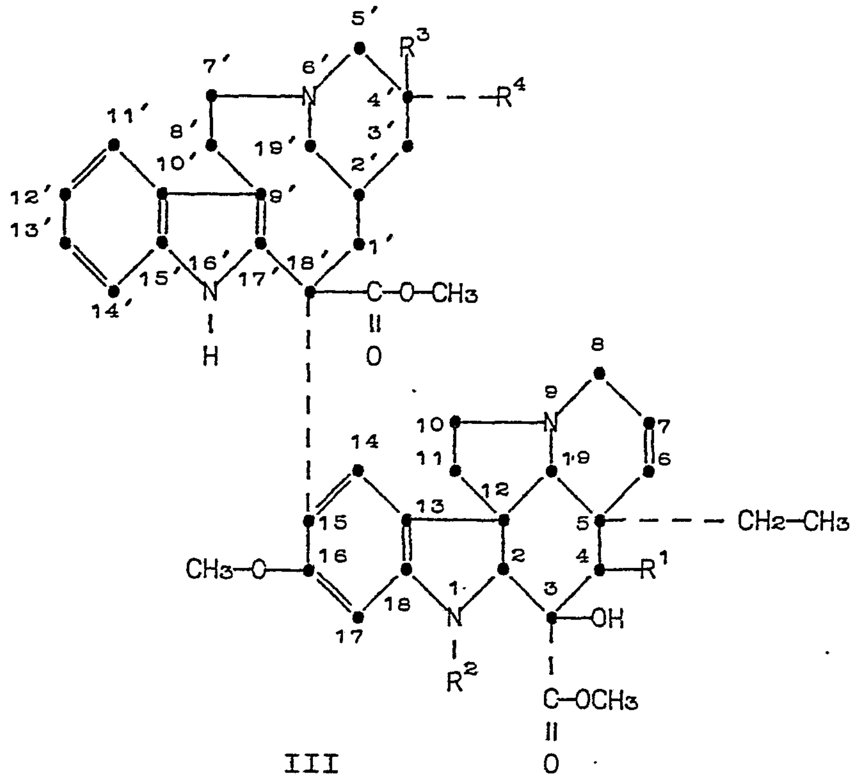

III

wherein $R^2$, $R^3$ and $R^4$ are as before defined and $R^1$ is $-O-CO-CH_3$; with an isocyanate of the formula:

$$OCN-CH_2-CH_2-S-R^7$$

wherein $R^7$ is $CH_3$ or $S-CH_2-CH_2-NCO$ to yield a thioethane of formula I wherein Q, $R^2$, $R^3$, and $R^4$ are as defined above, $R^1$ is $-O-CO-CH_3$, $R^6$ is $CH_3$ or $S-CH_2-CH_2-Z$ and Z is NCO; and optionally when $R^6$ is $S-CH_2-CH_2-Z$, Z is NCO and $R^1$ is $-O-CO-CH_3$, reacting said intermediate with a $(C_1-C_3)$alkanol to yield a thioethane of formula I wherein Q, $R^1$, $R^2$, $R^3$ and $R^4$ are as above and $R^6$ is $-SCH_2-CH_2-Z$ and Z is $-NHCO_2-(C_1-C_3)$alkyl; and optionally when $R^6$ is $-S-CH_2-CH_2-Z$

and Z is Q, NCO or $-NHCO_2(C_1-C_3)$alkyl provided that when Z is NCO $R^1$ is $-O-CO-CH_3$ reducing the compound to a compound wherein $R^6$ is H; and optionally when $R^6$ is H reacting with an oxidizing agent having an $E_o$ of about +.50 to prepare a compound wherein $R^6$ is $-S-CH_2-CH_2-Z$ and Z is Q; and optionally when $R^1$ is $-O-CO-CH_3$ and Z is Q or $NHCO_2$ $(C_1-C_3)$alkyl hydrolyzing any of the above intermediates with a mild alkali to prepare a compound wherein $R^1$ is OH; and recovering the product as a free base or as pharmaceutically-acceptable acid addition salt thereof.

The thioethanes of formula I wherein $R^6$ is $S-CH_2-CH_2-Z$ and Z is Q are prepared by reacting Vinca dimer of the formula III wherein $R^2$, $R^3$ and $R^4$ have

their previously assigned meaning and $R^1$ is -O-CO-$CH_3$, with an isocyanate of the structure OCN-$CH_2$-$CH_2$-S-$R^7$ to yield a thioethane of formula I in which $R^6$ is -$CH_3$, or an intermediate in which $R^6$ is -S-$CH_2$-$CH_2$-Z and Z is -N=C=O. When Z is NCO $R^1$ may not be OH. The compound wherein $R^6$ is -S-$CH_2$-$CH_2$-Z and Z is NCO is not isolated as such ordinarily but as a reaction product with a ($C_1$-$C_3$) alkanol having formula I wherein $R^6$ is -S-$CH_2$-$CH_2$-NH-CO-O-($C_1$-$C_3$)alkyl and $R^1$ is acetoxy.

Reduction of the thioethane of formula I wherein $R^6$ is S-S-$CH_2$-$CH_2$-Z and Z is Q, NCO or -NH-CO-O-($C_1$-$C_3$)alkyl and $R^1$ is -O-CO-$CH_3$ when Z is NCO, with zinc and acetic acid or other appropriate reducing agent yields the thioethane of formula I wherein $R^6$ is H. Oxidation of this thioethane of formula I with ferrocycanide or other suitable oxidizing agent having an $E_o$ of about +0.50 yields a thioethane of formula I wherein $R^6$ is -S-$CH_2CH_2$-Q.

In the above reaction involving a Vinca dimer III and an isocyanate, the secondary hydroxyl at C-4 is acetylated. A 4-hydroxy if present, would react with the isocyanate. It can be protected other than by acetylation, however.

It is believed that the formation of the oxazolidinedione ring starts with the reaction of the 3-hydroxy with an isocyanate to form a carbamate which, in a second step, cyclizes to an oxazolidinedione ring with loss of $CH_3OH$.

The thioethanes represented by I when $R^6$ is H, -$CH_3$ or S-$CH_2$-$CH_2$-Q are active antimitotic and

antineoplastic agents in their own right, but can also be converted by mild alkaline hydrolysis to carboxamide derivatives of VLB, vincristine, leurosidine etc. and of the corresponding 4-desacetyl compounds (structure III wherein the C-3 $\underset{\overset{\|}{O}}{C}-OCH_3$ group is replaced by a $\underset{\overset{\|}{O}}{C}-NH-CH_2-CH_2-S-CH_3$ or $\underset{\overset{\|}{O}}{C}-NH-CH_2-CH_2-SH$, and the corresponding disulfide--see Conrad et al., Jour. Med. Chem. 22 pages 391-400 (1979)--compounds 25, 26, 30 and 37 and United States patent 4,096,148, col. 7, line 32 et seq).

There is also disclosed a process for preparing an isocyanate of the formula $R^7-S-CH_2-CH_2-NCO$ wherein $R^7$ is $CH_3$ or $S-CH_2-CH_2-NCO$ which comprises reacting $R^8-S-CH_2-CH_2-NH_2$ wherein $R^8$ is $CH_3$ or $-S-CH_2-CH_2-NH_2$ with p-nitrophenylchloroformate to form the carbamate

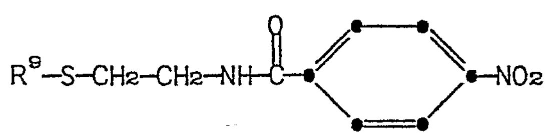

wherein $R^9$ is $CH_3$ or

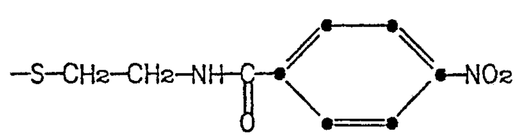

followed by reacting the carbamate with triethylamine.

This invention is further illustrated by the following specific examples.

## Example 1

Preparation of Vinblastine 3''-(β-[β-(methoxycarbonylamino)ethyldithio]ethyl)-3-spiro-5''-oxazolidine-2'',4''-dione.

A solution was prepared from 40.4 g. of p-nitrophenylchloroformate and 150 ml. of acetonitrile. This solution was added to a slurry of 22.5 g. of cystamine dihydrochloride and 150 ml. of anhydrous acetonitrile. The reaction mixture was heated to refluxing temperature for about 2 days after which time it was cooled and filtered. The filter cake comprising cystamine-bis-carbamate was crystallized from hot toluene. The crystalline product was then stirred with water for one-half hour to remove unreacted cystamine. This aqueous mixture was filtered and the precipitate washed with water. Yield of cystamine-bis(p-nitrophenyl)carbamate was 19.5 g. melting at 163-165°C.

nmr (DMSO-$d_6$): 2.91 (4H, t), 3.46 (4H, t), 3.82 (2H bis, exchangeable with $D_2O$), 7.41 (4H, d), 8.26 (4H, d) δ.

Infrared spectrum (mull); 3360, 1710, 1350, 1215 $cm^{-1}$.

Mass spectrum: m/e 204 (loss of carbamate from molecular ion plus fragmentation of the disulfide bond).

Analysis Calculated for: $C_{18}H_{18}N_4O_8S_2$:

Theory: C, 44.81; H, 3.76; N, 11.61; S, 13.29.

Found: C, 45.05; H, 3.97; N, 11.80; S, 13.55.

Seven and nine-tenths grams of cystamine-bis(p-nitrophenyl)carbamate and 2.9 g. of triethylamine were dissolved in toluene and the resulting solution heated to 40°C. This solution was added in dropwise fashion to a solution of 3.1 g. of trimethylsilylchloride in 25 ml. of toluene. The resulting mixture was heated to 90°C. for about 3 hours, and was then cooled. Triethylamine hydrochloride precipitated and was separated by filtration. The filtrate, containing cystamine-bis-isocyanate formed in the above reaction, was concentrated to about 20 ml. 4.8 G. of vinblastine free base were dissolved in the toluene solution containing the isocyanate. This reaction mixture was heated to about 60°C. for about 10 hours, followed by a heating period of 3 hours at about 80°C. Volatile constituents of the reaction mixture were then removed by evaporation in vacuo leaving 13.7 g. of an oil. This residual oil was chromatographed over 475 g. of silica gel (Woelm activity I). The following eluants were used, based on a 1:1 mixture of methylenedichloride and ethylacetate: 4 l. containing 5% methanol, one l. containing 7.5% methanol, 2 l. containing 10% methanol and 3 l. containing 15% methanol. Fractions shown by tlc to contain vinblastine 3''-(β-[β-(methoxycarbonylamino)ethyldithio]ethyl)-3-spiro-5''-oxazolidine-2'',4''-dione were combined and the solvent removed from the combined products in vacuo. Purified vinblastine 3''-(β-[β-(methoxycarbonylamino)ethyldithio]-ethyl)-3-spiro-5''-oxazolidinedione thus prepared (1.24 g.) had the following physical characteristics:

Mass spectrum: m/e 895, 882, 865, 835, 804, 792, 649, 343, 525, 494, 355, 154; field desorption 1014, 882.

nmr ($CDCl_3$): .68 (3H, t), 0.89 (3H, t), 2.02 (3H, s, NMe), 3.60 (3H, s, $CO_2Me$), 3.66 (4H, $CH_2CH_2S$), 3.79 (3H, s, MeOAr), 5.26 (1H, m, C-6), 5.41 (1H, s, C-4), 5.89 (1H, m, C-7), 6.11 (1H, s, C-17), 6.65 (1H, s, C-14), 7.2-7.5 (4H, m, Ar), 8.15 (1H, br s, NH) $\delta$.

Infrared spectrum: 3600, 3460, 1814 (oxazolidinedionecarbonyl), 1745, 1725, 1700 $cm^{-1}$.

cmr ($CDCl_3$): 154.6, 157.1, 169.8 $\delta$ (new carbonyls due to oxazolidinedione carbamate)

## Example 2

Preparation of Vinblastine 3''-(β-thioethyl)-3-spiro-5''-oxazolidine-2'',4''-dione

A solution was prepared from 507.1 mg. of vinblastine 3''-(β-[β-(methoxycarbonylamino)ethyldithio]ethyl)-3-spiro-5''-oxazolidine-2'',4''-dione sulfate and 20 ml. of glacial acetic acid. 317.7 Mg. of zinc dust were added and the resulting mixture heated to refluxing temperature for about 1.5 hours. At this time, tlc showed that the reaction had gone essentially to completion. Ice was added to the reaction mixture followed by dilute aqueous ammonium hydroxide to pH = 8. Water was added and the resulting aqueous mixture extracted 6 times with an equal volume of methylenedichloride. The methylenedichloride extracts were combined and filtered and the solvent removed from the filtrate by evaporation *in vacuo*. The

resulting residue was dried with a toluene azeotrope. Yield of vinblastine 3''-(β-thioethyl)-3-spiro-5''-oxazolidine-2'',4''-dione thus prepared was 391.9 mg. The compound had the following characteristics:

nmr ($CDCl_3$): 0.67 (3H, t), 0.88 (3H, t), 2.01 (3H, s), 2.81 (m), 2.84 (3H, s), 3.61 (3H, s), 3.68 (s), 3.79 (3H, s), 4.0 (m), 5.18 (1H, m), 5.45 (1H, s), 5.88 (1H, s), 6.10 (1H, s), 6.64 (1H, s), about 7.5 (4H, m), 8.12 (1H, brs), δ.

Mass spectrum: m/e 895 (transmethylation), 881, 864, 850, 822, 820, 792, 650, 543, 381, 354, 154, 135.

Infrared spectrum ($CHCl_3$): 3670, 3590, 3450, 1815, 1740, 1715, 1615 $cm^{-1}$.

$pK_a$ in 66% aqueous DMF = 5.2, 7.0, 11.1.

The product was converted to the sulfate salt by standard procedures.

## Example 3

Preparation of Bis-[β-(vinblastine-3-spiro-5''-oxazolidine-2'',4''-dione-3''-yl)ethyl]disulfide

A solution containing 327.6 mg. of vinblastine 3''-(2-thioethyl)-3-spiro-5''-oxazolidine-2'',4''-dione free base in about 2 ml. of water was prepared by adding sufficient 12N aqueous hydrochloric acid to solubilize the base. 375.1 Mg. of potassium ferricyanide were added. There was an immediate heavy percipitate. The reaction mixture was diluted with 30 ml. of water and the pH adjusted to about 8 by the addition of 14N aqueous ammonium hydroxide. The resulting aqueous mixture was extracted 5 times with an equal volume of

methylenedichloride. The methylenedichloride extracts were combined. The combined extracts were concentrated by evaporation in vacuo and dried with a toluene azeotrope. The residue containing bis-[β-vinblastine-3-spiro-5''-oxazolidine-2'',4''-dione-3''-yl)ethyl]-disulfide (weight = 331.7 mg.) was chromatographed over 35 g. of silica (Woelm activity 1). The chromatogram was developed by employing 300 ml. fractions of a 1:1 ethylacetate-methylenedichloride solvent mixture containing increasing amounts of methanol from 4% up to 45%. Fractions shown by tlc to contain the desired disulfide ($R_f$ = 0.25 in a 3:1 ethylacetate-ethanol solvent mixture) were combined and the solvent removed: yield = 55.9 mg. The compound had the following physical properties:

nmr ($CDCl_3$): 0.68 and 0.89 (12H, 2t), 2.03 (6H, s, OAc), 2.85 (6H, s, NMe), 5.08 (2H, m), 6.11 (2H, s), 6.65 (2H, s), 7.1-7.5 (4H, m, Ar), 8.21 (2H, br s) δ.

Infrared spectrum ($CHCl_3$): 3680, 3600, 3460, 1815, 1745, 1720 (shoulder) $cm^{-1}$.

Mass spectrum: m/e 835 (cleavage of disulfide and loss of $CH_2S$), 817, 804, 775, 592, 494, 355, 154; field desorption 881.

Osmometric weight determination = 2275 (theoretical 1760).

The sulfate salt was prepared by the usual procedure.

Example 4

Preparation of Vinblastine 3''-1β-methylmercaptoethyl)-3-spiro-5''-oxazolidine-2'',4''-dione.

β-Methylmercaptoethyl isocyanate was prepared by the process of preparing cystamine-bis-isocyanate described in Example 1. VLB, free base, 1.03 g, was reacted with an excess of the isocyanate to prepare VLB 3''-(β-methylmercaptoethyl)-3-spiro-5''-oxazolidine-2'',4''-dione by the process described in Example 1. Yield after chromatographic purification was 374 mg. The physical characteristics of the purified material thus prepared were:

Mass spectrum: m/e 895, 864, 834 & 6, 792.

NMR ($CDCl_3$): .70 (t), .92 (t), 2.04 (s, OAc), 2.15 (s, SMe), 2.87 (s, NMe), 3.62 (s, $CO_2$MeTop), 3.80 (s, MeOAr)

Infrared spectrum ($CHCl_3$): 3660, 3590, 3450, 1810, 1735, 1610, 1495, 1450, 1435 & 1425, 1405, 1365, 1325.

Example 5

Salts of the thioethanes of formula V are prepared as follows:

The free base is dissolved in a minimal quantity of ethanol. The pH is lowered to 4.0 ± 0.2 by dropwise addition of 2 percent sulfuric acid in ethanol. The pH is determined by taking a 2-drop aliquot, diluting the drop to 1 ml. with water and then determining the pH using a pH meter. The acidic ethanolic solution is evaporated to dryness. The residue comprising the sulfate salt can be recrystallized from methanol or ethanol if desired.

As previously stated, the compounds of this invention are antimitotics, and can adversely affect the growth of malignant cells. This activity is manifested in a standard mitotic inhibition test employing Chinese hamster ovary cells.

Table 1 gives the results of this test for the compounds of this invention. In the Table, column 1 gives the name of the compound, column 2 gives the dose in mcg/ml for accumulation in mitosis and column 3, the percent mitotic accumulation as a range.

Table 1

| Name of Compound | Dose | Range |
|---|---|---|
| Vinblastine 3''-(β-(β-methoxycarbonylamino)ethyldithio]ethyl)-3-spiro-5''-oxazolidine-2'',4''-dione | 2,20 | 7-10 |

Table 1 (cont'd)

| Name of Compound | Dose | Range |
|---|---|---|
| vinblastine 3''-(β-thioethyl)-3-spiro-5''-oxazolidine-2'',4''-dione | 2,20 | 10-15 |

The bases of this invention and their salts preparable by the processes disclosed herein are active also in vivo against some but not all transplanted tumors in mice. To demonstrate such activity, a protocol was used involving the administration of the drug by the intraperitoneal or oral route, at selected dose levels, against $6C_3HED$ lymphosarcoma, B16 melanoma, 755 adenocarcinoma and $C_3H$ mammary carcinoma.

The following table - Table 2 - gives the results of this experiment in which mice bearing the transplanted tumor were treated with a compound of this invention. In the table, column 1 gives the name of the compound; column 2, the name of the tumor; column 3, the dosage given; and column 4, the percent inhibition of tumor growth (I) or prolongation of life (PL). The following dosage regimen was employed: every fourth day (three doses) starting on the first day after inoculation.

Table 2

| Name of Compound | Tumor | Dose (mg/kg) | Percent Inhibition or Prolongation |
|---|---|---|---|
| vinblastine 3''-(β-[β-(methoxycarbonylamino)ethyldithio]-ethyl)-3-spiro-oxazolidine 2'',4''-dione | $6C_3HED$ | 2.5 | 12*(I) |
| | P1534(J) | 2.5 | 16*(I) |
| | P388/V | 2.5 | 38(PL) |
| | | 2.0 | 25*(PL) |
| | | 1.5 | 30(PL) |
| vinblastine 3''-(β-thioethyl)-3-spiro-5''-oxazolidine-2'',4''-dione | $6C_3HED$ | 2.5 | 46*(I) |
| | P1534(J) | 2.5 | 16*(I) |
| | P388/V | 2.5 | 18*(PL) |
| bis-[β-(vinblastine 3-spiro-5''-oxazolidone-2'',4''-dione-3''-yl)ethyl]disulfide | $6C_3HED$ | 2.5 | 14(I) |
| | P1534(J) | 2.5 | 14*(I) |
| | P388/J | 2.5 | 22*(PL) |
| vinblastine 3''-(β methylmercaptoethyl)-3-spiro-5''-oxazolidine-2'',4''-dione | $6C_3HED$ | 2.5 | 45(I) |
| | P388 | 1.5 | 43(PL) |
| 4-desacetyl vinblastine 3''-(β-thioethyl)-3-spiro-5''-oxazolidine-2'',4''-dione | P388 | 1.4 | 77(PL) |
| | | 1.2 | 65(PL) |
| | | 1.0 | 52(PL) |

In the above Table, $6C_3HED$ is a lymphosarcoma and P1534(J) and P388/J are leukemias.

*Percentages below 30 are not statistically significant.

In utilizing the novel compounds of this invention as anti-tumor agents in mammals, either the parenteral or oral route of administration may be employed. For such use, the drug is customarily mixed with a pharmaceutically suitable carrier. With parenteral administration, the intravenous route is preferred although, with smaller mammals such as mice, the intraperitoneal route may be used. For intravenous administration, isotonic solutions containing about 1 mg./ml. of a salt of an alkaloidal base of formula II above are employed. The drug is administered at a dose of from 0.01 to 10 mg./kg. of mammalian body weight once or twice a week or every two weeks depending on both the activity and the toxicity of the drug. An alternative method of arriving at a therapeutic dose is based on body surface area with a dose in the range 0.1 to 10 mg./meter squared of mammalian body surface administered every 7 or 17 days or twice weekly.

As would be expected, the compounds of this invention differ in their anti-tumor spectrum from that of VLB, vincristine and vindesine in the same way that the anti-tumor spectra of those compounds differ among themselves, one drug being more effective against certain tumors or classes of tumors and less effective against others. However, in utilizing the compounds of this invention clinically, an oncologist would administer one of them initially by the same route in the same vehicle and against the same types of tumors as employed clinically with vindesine, vincristine and VLB. Differences in dosage level would, of course, be based on relative oncolytic potency and toxicity.

## Claims

1. A pharmaceutical formulation which comprises as active ingredient a thioethane of formula I

$$Q\text{-}CH_2\text{-}CH_2\text{-}S\text{-}R^6 \qquad I$$

wherein Q is a 3"-oxazolidinedionyl radical of the formula II:

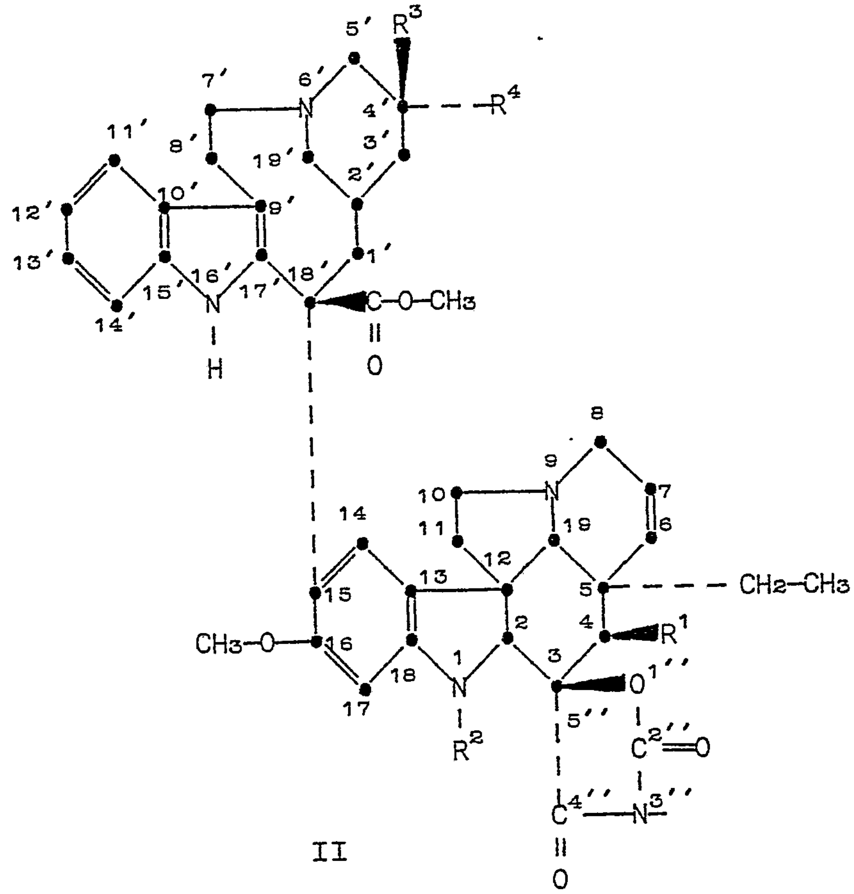

II

wherein $R^1$ is -OH or $-O-CO-CH_3$; $R^2$ is CHO or $CH_3$; one of $R^3$ and $R^4$ is OH or H and the other is ethyl; and $R^6$ is H, $CH_3$ or $S-CH_2-CH_2-Z$;

Z is Q, NCO or $NH-CO_2-(C_1-C_3)$alkyl, provided that when Z is NCO, $R^1$ is $-O-CO-CH_3$; or a pharmaceutically-acceptable acid addition salt thereof associated with at least one carrier or diluent therefor.

2. A thioethane of formula I as defined in claim 1 with the proviso that when the compound is a free base $R^6$ is not H.

3. A thioethane of formula I as claimed in claim 2 selected from the group:

vinblastine 3''-(β-[β-(methoxycarbonylamino)-ethyldithio]ethyl)-3-spiro-oxazolidine-2'',4''-dione,

vinblastine 3''-(β-thioethyl)-3-spiro-5''-oxazolidine-2'',4''-dione, sulfate salt

bis-[β-(vinblastine 3-spiro-5''-oxazolidinone-2'',4''-dione-3''-yl)ethyl]disulfide,

vinblastine 3''-(β-methylmercapto)-3-spiro-5''-oxazolidine-2'',4''-dione, and

vinblastine 3''-(β-[β-isocyanatoethyldithio]-ethyl)-3-spiro-5''-oxazolidine-2'',4''-dione.

4. An isocyanate of the formula $OCN-CH_2-CH_2-S-R^7$ wherein $R^7$ is $CH_3$ or $S-CH_2-CH_2-NCO$.

5. A process for preparing a β-thioethyl compound of formula I as defined in claim 1 wherein Q, $R^1$, $R^2$, $R^3$, $R^4$ and Z are as before defined, provided that when Z is NCO, $R^1$ is $-O-CO-CH_3$; which comprises reacting a compound of the formula III

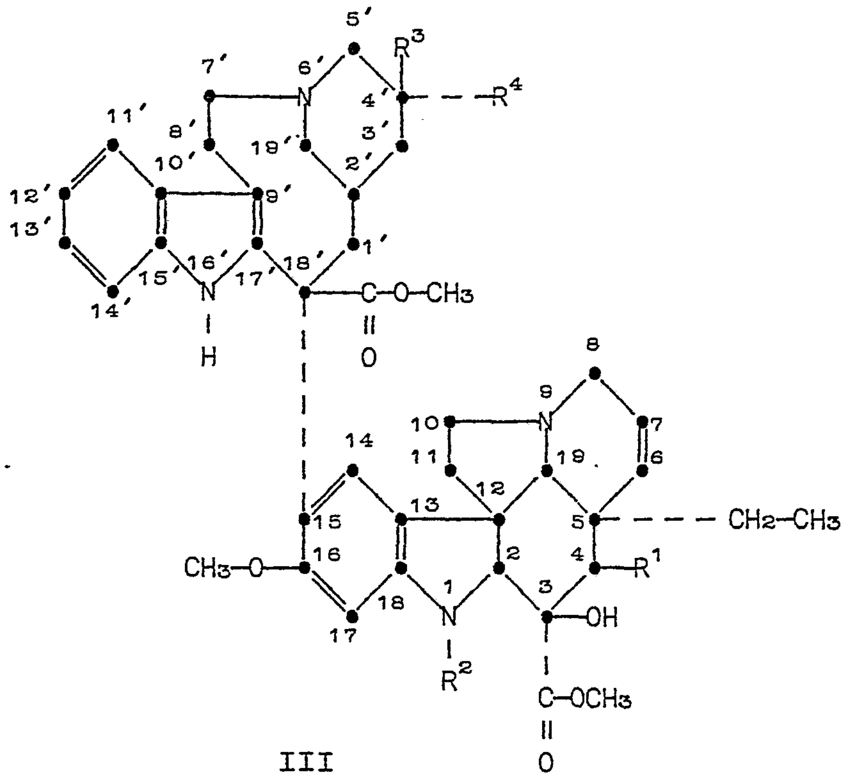

III

wherein $R^2$, $R^3$ and $R^4$ are as before defined and $R^1$ is $-O-CO-CH_3$; with an isocyanate of the formula:

$$OCN-CH_2-CH_2-S-R^7$$

wherein $R^7$ is $CH_3$ or $S-CH_2-CH_2-NCO$ to yield a thioethane of formula I wherein Q, $R^2$, $R^3$, and $R^4$ are as defined above, $R^1$ is $-O-CO-CH_3$, $R^6$ is $CH_3$ or $S-CH_2-CH_2-Z$ and Z is NCO; and optionally when $R^6$ is $S-CH_2-CH_2-Z$, Z is NCO and $R^1$ is $-O-CO-CH_3$ reacting said intermediate with a $(C_1-C_3)$alkanol to yield a thioethane of formula I wherein Q, $R^1$, $R^2$, $R^3$ and $R^4$ are as above and $R^6$ is $-SCH_2-CH_2-Z$ and Z is $-NHCO_2(C_1-C_3)$-alkyl; and optionally when $R^6$ is $-S-CH_2-CH_2-Z$ and Z is Q, NCO or $-NHCO_2(C_1-C_3)$alkyl provided that when Z is

NCO $R^1$ is $-O-CO-CH_3$ reducing the compound to a compound wherein $R^6$ is H; and optionally when $R^6$ is H reacting with an oxidizing agent having an $E_o$ of about +.50 to prepare a compound wherein $R^6$ is $-S-CH_2-CH_2-Z$ and Z is Q; and optionally when $R^1$ is $-O-CO-CH_3$ and Z is Q or $-NHCO_2(C_1-C_5)$alkyl hydrolyzing any of the above intermediates with a mild alkali to prepare a compound wherein $R^1$ is OH; and recovering the product as a free base or as pharmaceutically-acceptable acid addition salt thereof.

6. A process for preparing a thioethane of formula I as claimed in claim 5 wherein Q, $R^1$, $R^2$, $R^3$, $R^4$ are as above, $R^6$ is $-S-CH_2-CH_2-Z$ and Z is Q which comprises reacting a thioethane of formula I wherein all substituents are as above except $R^6$ is H with an oxidizing agent having an $E_o$ of about +0.50; and optionally, when $R^1$ is $-O_2CCH_3$ hydrolyzing the above intermediate with a mild alkali to prepare a compound wherein $R^1$ is OH.

7. A process for preparing a thioethane of formula I as claimed in claim 5 wherein Q, $R^1$, $R^2$, $R^3$ and $R^4$ are as defined above, and $R^6$ is hydrogen which comprises reducing a thioethane compound of formula I wherein $R^6$ is $-S-CH_2-CH_2-Z$ and Z is NCO, $NHCO_2(C_1-C_3)$ alkyl or Q and providing that when Z is NCO, $R^1$ is $-O-CO-CH_3$; and optionally, when $R_1$ is $-O-CO-CH_3$ reacting any of the above intermediates with a mild alkali to prepare a compound wherein $R^1$ is -OH.

8. A thioethane of formula I as defined in claim 1 for use in the therapeutic treatment of a mammal which is suffering from a tumor susceptible thereto.

9. A process for preparing an isocyanate of the formula $R^7$-S-$CH_2$-$CH_2$-NCO wherein $R^7$ is $CH_3$ or S-$CH_2$-$CH_2$-NCO which comprises reacting $R^8$ -S-$CH_2$-$CH_2$-$NH_2$ wherein $R^8$ is $CH_3$ or -S-$CH_2$-$CH_2$-$NH_2$ with p-nitrophenyl-chloroformate to form the carbamate

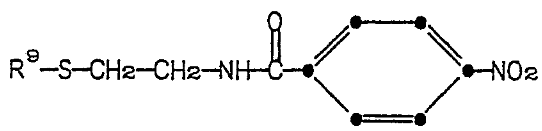

wherein $R^9$ is $CH_3$ or

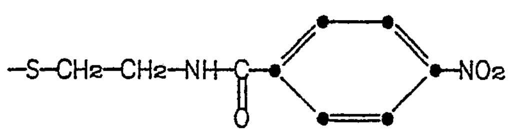

followed by reacting the carbamate with triethylamine.

10. An isocyanate of the formula $R^7$-S-$CH_2$-$CH_2$-NCO as claimed in claim 4 for use in preparing a thioethane of formula I as described in claim 1.